# FASCICULE DE BREVET EUROPEEN

(11) **EP 0 467 776 B1**
(45) Date de publication et mention de la délivrance du brevet: **28.09.1994**
(21) Numéro de dépôt: 91401986.4
(22) Date de dépôt: 16.07.1991
(51) Int. Cl.: A61K 9/12, A61K 9/46, A23P 1/16, A61K 7/48

(54) **Nouvelles formes pharmaceutiques, leur procédé de préparation et leurs applications**
Arzneiformen, deren Herstellungsverfahren sowie deren Anwendungen
Pharmaceutical forms, their process of preparation and their uses

(30) Priorité: 16.07.1990 FR 9009464
(43) Date de publication de la demande: 22.01.1992
(73) Titulaire: Dubois, Jacques, F-11100 Narbonne (FR)
(72) Inventeur: Dubois, Jacques, F-11100 Narbonne (FR)
(74) Mandataire: Morelle, Guy Georges Alain

(56) Documents cités:
- EP-A- 0 153 836
- EP-A- 0 286 085
- EP-A- 0 358 959
- EP-A- 0 362 655
- FR-A- 1 195 918
- LU-A- 28 065
- US-A- 2 274 252
- US-A- 3 594 467
- DERWENT FILE SUPPLIER WPIL, 1983, AN=83-749328 [35], Derwent Publications Ltd,Londres, GB; & JP-A-58 121 766 (AJINOMOTO K.K.) 20-07-1983

## Description

La présente invention est relative à de nouvelles formes pharmaceutiques, à leur procédé de préparation et à leurs applications dans les secteurs thérapeutique, diététique, hygiénique et dermo-cosmétologique.

Les nouvelles formes, objet de l'invention correspondent à des mousses obtenues extemporanément. Ces mousses, absorbables par la voie orale en raison de leur parfaite innocuité et de leur valeur diététique ou administrée en application sur la peau et les muqueuses, peuvent être utilisées telles quelles pour leurs propriétés ou servir d'excipient joint à un principe actif adéquat.

Le procédé de préparation de ces mousses est caractérisé par l'emploi combiné de deux constituants de base :
- un agent viscosant ou liant,
- un mélange effervescent.

Ces constituants étant mélangés à sec, la mousse est obtenue au moment de son utilisation par simple addition d'eau et/ou d'alcool ou solvant volatil qui déclenche simultanément deux phénomènes indépendants qui se combinent pour permettre l'obtention de la mousse:
- d'une part, la libération de gaz carbonique et,
- d'autre part, l'augmentation de la viscosité du milieu.

En effet, par agitation, le gaz carbonique libéré est réparti dans la masse et permet de former un gel dans lequel les bulles gazeuses sont retenues pour constituer instantanément une structure alvéolaire et poreuse.

L'agent viscosant ou liant est un constituant capable, en présence d'eau ou d'alcool ou d'un mélange eau-alcool, d'accroître la viscosité du milieu ou d'agir comme agglutinant -liant en milieu poreux. De nombreux agents viscosants et/ou liants sont disponibles ; certains sont d'origine naturelle, soit végétale (pectines, galacto-mannanes, gommes, alginates, gelose, cellulose, mucilages naturels divers), soit animale ou biologique (gelatine, dextranes, xanthanes, protéines du lait ...) ou chimique (methyl cellulose, carboxymethyl cellulose, hydroxy dérivés de la celulose, polyvinylpyrrolidone ...).

Toutes ces substances sont susceptibles de former des mousses. Les meilleurs résultats ont été cependant obtenus avec la gélatine, l'alginate de magnésium, la pectine, la gomme guar et les mucilages naturels, par suite de leur plus grande vitesse de gonflement et de viscosité.

Tous ces constituants peuvent bien évidemment être utilisés seuls ou en combinaison.

Des résultats inattendus et exceptionnellement intéressants ont été obtenus avec un constituant qui n'est habituellement pas considéré comme un agent viscosant : il s'agit des protéines coagulables du lait. Ces protéines présentent en effet un optimum des caractéristiques requises pour former une mousse d'excellente qualité. En outre, leur goût et leur valeur nutritive leur confère un avantage supplémentaire.

Le terme générique de "protéines coagulables du lait" recouvre l'ensemble des substances obtenues par coagulation du lait provoquée par tout procédé : enzymatique, chimique ou thermique.

Il est bien connu que le composant principal de ces protéines coagulables du lait est la caséine. Cette protéine est, en l'état actuel de l'économie, un sous-produit de l'industrie laitière. Elle est disponible en grandes quantités, notamment comme sous-produit de la préparation des fromages et des produits du type yaourts.

Le lactosérum constitue environ 3,3 % du poids total du lait. Il est constitué d'un mélange de caséine, de lactalbumine et de lactoglobuline. La caséine est le constituant principal de ce mélange de protéines.

Il faut noter accessoirement que la caséine peut être obtenue à partir de produits végétaux, et notamment de graines de soja.

La présence de caséine contribue à la stabilité de l'émulsion naturelle que constitue le lait. Elle possède des propriétés émulsionnantes douces qui permettent de disperser les globules de graisse au sein du lactosérum. En outre, nous rappellerons qu'elle constitue la seule source de protéines alimentaires complètes indispensables à la croissance du nourrisson.

Elle est disponible soit sous forme de solution de lactosérum ou de solutions aqueuses de sels alcalins ou alcalino-terreux de caséine (sels de sodium, potassium, calcium ou sels mixtes notamment) soit sous forme de poudres obtenues par séchage ou atomisation de ces solutions.

Il est nécessaire de préciser que toutes ces sources de caséine sont utilisables, mais que les résultats les plus satisfaisants sont obtenus avec la caséine sèche, pour des raisons de propreté microbiologique et pour des raisons de conservation essentiellement, et sous forme rapidement dispersible. Cette caractéristique implique à la fois :
- une forte concentration en protéines, au delà de 70 % et si possible 95%,
- une finesse de la poudre utilisée, qui doit pouvoir se mélanger avec les autres constituants et gonfler rapidement dans l'eau,
- une aptitude à la dispersion dans l'eau obtenue au plus haut point avec les caséines solubles telles que la caséine potassique, la caséine sodique, la caséine calcique ou les produits salifiés mixtes.

Les meilleurs résultats lors des esssais ont été obtenus avec la caséine sodique, ou la caséine sodico-calcique, en poudre fine, de granulométrie inférieure à 500 »m.

Le deuxième constituant de base est le mélange effervescent. Sa fonction est de produire, par simple contact avec l'eau, de manière instantanée, rapide et prolongée, du gaz carbonique. Les produits de ce type sont bien connus : ils sont classiquement obtenus par l'association d'un composé acide et d'un composé alcalin. Parmi les plus connus on peut citer les acides citrique, tartrique, lactique ... et les bases ou sels alcalins (bicarbonates et/ou carbonates de métaux alcalins ou alcalino-terreux).

Parmi toutes les combinaisons possibles, on citera à titre d'exemple le mélange acide tartrique-carbonate acide de sodium à des concentrations comprises entre 0,1 % et 40 % en fonction de la formulation et de la concentration en agent viscosant, et plus particulièrement entre 0,5% et 20 %.

D'autres constituants peuvent être introduits selon l'objectif particulier que l'on cherche à atteindre.

Ainsi, la lécithine de soja peut être avantageusement utilisée : en effet, à des doses comprises entre 0,1 % et 30 %, et plus particulièrement entre 0,2 % et 10 %, elle améliore considérablement la dispersibilité dans l'eau de l'agent viscosant-liant : rendant sensiblement plus rapide son mouillage par l'eau. Elle permet de produire une mousse de bonne qualité en favorisant la synchronisation des deux phénomènes essentiels : la prise en gelée de la phase aqueuse et le dégagement de gaz carbonique.

D'autres agents peuvent encore intervenir et produire un effet différent selon la concentration à laquelle ils sont utilisés : il s'agit des hydrates de carbone (saccharose, glucose, fructose, mannitol, sorbitol, amidons modifiés ...), des acides aminés (glucocolle, lysine, ...) des hydrolysats de protéines animales ou végétales. A faible dose, ces composés pourront servir de diluant ou de ballast. A forte dose, ils agiront eux-même comme agents liants ou agglutinants. Enfin, possédant une saveur douce ou sucrée, ils amélioreront le goût du produit fini.

Il faut bien évidemment signaler que tous les composants une fois mélangés, la poudre obtenue est aussitôt conditionnée en des sachets unitaires à l'abri de l'air et de l'humidité.

Le procédé de l'invention permet de réaliser des mousses extemporanées. Il s'agit des mousses du type décrit précédemment. Quelques exemples, intéressant les domaines de la diététique et de la pharmacie, sont donnés ci-après à titre illustratif :

### a) Domaine diététique

### Exemple No. 1

Le produit de base constitue en lui-même un apport nutritionnel particulièrement estimable, ainsi qu'il apparaît dans la formule n° 1 citée à titre d'exemple non limitatif

| | |
|---|---|
| caséine sodico-calcique | 25, 0 g |
| extrait de lécithine | 10,0 g |
| sucre cristallisé | 10, 0 g |

| mélange effervescent | |
|---|---|
| acide citrique | 2,5 g |
| carbonate acide de sodium | 4, 0 g |
| arôme, talc | Q.S |

Après agitation avec 80 ml d'eau, on obtient environ 130 g de mousse dont la valeur nutritive tient à la présence de protides assimilables et complets en acides aminés et de glucides à action rapide.

Elle peut être utilisée en particulier chez les dénutris (enfants ou vieillards) ou par les sportifs comme reconstituant musculaire.

Une autre application dans le domaine diététique est constituée par l'addition, à la formule de base de divers principes actifs tels que vitamines, sels minéraux, acides aminés ...,

### Exemple No. 2

| | |
|---|---|
| protéines coagulables du lait | 4,0 g |
| extrait de lécithine | 0,8 g |
| glucose | 3,0 g |

| mélange effervescent | |
|---|---|
| acide tartrique anhydre | 0,4 g |
| carbonate acide de sodium | 0,5 g |
| talc | 0,3 g |
| lysine | 0,3 g |
| méthionine | 0,3 g |
| cystéine | 0,3 g |

Il faut préciser que certains constituants peuvent être amenés dans le diluant aqueux.

### b) Domaine pharmaceutique

La mousse extemporanée peut servir de véhicule capable de faciliter l'administration de substances médicamenteuses dont le goût ou l'aspect peuvent entraîner un refus, notamment chez les enfants ou les personnes âgées : elle est en effet agréablement et facilement ingérable. Il est ainsi possible d'ajouter le principe actif à la formule de base : on peut ainsi assurer aux substances instables en milieu aqueux une longue conservation jusqu'à son utilisation.

Une autre possibilité est de mettre la formule de base à la disposition du malade lui permettant d'effectuer lui-même le mélange avec la préparation médicamenteuse à ingérer.

### Exemple No. 3 : Formule de base

| | |
|---|---|
| alginate de magnésium | 1,3 g |
| extrait de lécithine | 0,4 g |
| glucose anhydre | 2,0 g |

| mélange effervescent | |
|---|---|
| acide lactique anhydre | 0,3 g |
| carbonate acide de sodium | 0,3 g |
| talc | 0,15 g |

Par addition de 8 ml d'eau ou de soluté médicamenteux, cette formule peut contenir jusqu'à 1g de principe actif en poudre (antibiotique, antifungique, antiinflammatoire ...)

### Exemple No. 4 : Formule de base

| | |
|---|---|
| pectine | 0,3 g |
| fructose | 1,0 g |

| mélange effervescent | |
|---|---|
| acide tartrique anhydre | 0,25 g |
| carbonate acide de sodium | 0,40 g |
| arôme | Q.S |

Cette formule peut être additionnée de 0,5 g de prinicpe actif, par exemple d'acide acétylsalicylique (ou d'un sel de sodium ou de lysine), paracétamol, prométhazine...

Une autre application des mousses de l'invention est la préparation de produits destinés à être appliqués sur la peau et les muqueuses.

La mousse peut ainsi être additionnée de différents principes actifs adaptés à chaque besoin particulier et permettre ainsi l'utilisation de substances instables en milieu aqueux ou l'association de principes actifs incompatibles.

Pour cette utilisation, il est possible d'incorporer des agents surgraissants (extrait de lécithine par exemple).

Il faut enfin signaler que cette préparation peut être présentée dans des conditionnements mettant en valeur la commodité d'emploi du produit en ambulatoire en associant au besoin la poudre sèche avec un diluant choisi, par exemple la L-leucine, permettant ainsi la combinaison de plusieurs principes actifs et la réalisation commode du mélange.

## Revendications (Revendications pour l'(les) Etat(s) contractant(s) suivant(s): AT, BE, CH, LI, DE, DK, GB, IT, LU, NL, SE)

1. Procédé de préparation de mousses extemporanées utilisables en thérapeutique, diététique, dermo-cosmétologie, dermatologie et hygiène, ***caracterisé en ce qu'*** une poudre sèche de conservation prolongée, composée de caséine et d'un mélange effervescent est mise à réagir à froid avec un diluant.

2. Procédé selon la Revendication 1, ***caracterisé en ce que*** la caséine est additionnée de lécitihine dans une proportion comprise entre 0,1 et 30 %.

3. Procédé selon les Revendications 1 ou 2, ***caracterisé en ce que*** l'agent effervescent est constitué par l'association d'un composé acide et d'un composé alcalin.

4. Procédé selon les Revendications 1 ou 2, ***caracterisé en ce que*** le diluant est soit l'eau soit un mélange eau/alcool, soit l'alcool pur.

5. Préparation susceptible d'être obtenue selon de procédé de l'une quelconque des Revendications 1 à 4, ***caracterisée en ce qu'*** elle est présentée sous forme de poudre.

6. Préparation selon la Revendication 5, ***caracterisée en ce qu'***elle est présentée sous une forme appropriée à l'administration orale en usage thérapeutique ou diététique.

7. Préparation selon la Revendication 5, ***caracterisée en ce qu'***elle est présentée sous une forme appropriée à des applications externes dans le domaine de l'hygiène, de la dermatologie et de la dermocosmétologie.

## Revendications (Revendications pour l'(les) Etat(s) contractant(s) suivant(s): ES, GR)

1. Procédé de préparation de mousses extemporanées utilisables en thérapeutique, diététique, dermo-cosmétologie, dermatologie et hygiène, ***caracterisé en ce qu'***une poudre sèche de conservation prolongée, composée de caséine et d'un mélange effervescent est mise à réagir à froid avec un diluant.

2. Procédé selon la Revendication 1, ***caracterisé en ce que*** la caséine est additionnée de lécitihine dans une proportion comprise entre 0,1 et 30 %.

3. Procédé selon les Revendications 1 ou 2, ***caracterisé en ce que*** l'agent effervescent est constitué par l'association d'un composé acide et d'un composé alcalin.

4. Procédé selon les Revendications 1 ou 2, ***caracterisé en ce que*** le diluant est soit l'eau soit un mélange eau/alcool, soit l'alcool pur.

## Claims (Claims for the following Contracting State(s): AT, BE, CH, LI, DE, DK, GB, IT, LU, NL, SE)

1. Process for preparing foams which are obtained at the time of use and which can be used in therapy, dietetics, dermocosmetology, dermatology and hygiene, characterized in that a dry powder having an extended storage life, composed of casein and an effervescent mixture, is reacted in the cold state with a diluent.

2. Process according to Claim 1, characterized in that lecithin is added to the casein, in a proportion of between 0.1 and 30 %.

3. Process according to Claim 1 or 2, characterized in that the effervescent agent consists of the combination of an acidic compound and an alkaline compound.

4. Process according to Claim 1 or 2, characterized in that the diluent is either water or a water/alcohol mixture or pure alcohol.

5. Preparation capable of being obtained according to the process of any one of Claims 1 to 4, characterized in that it is presented in powder form.

6. Preparation according to Claim 5, characterized in that it is presented in a form suitable for oral administration in therapeutic or dietary application.

7. Preparation according to Claim 5, characterized in that it is presented in a form suitable for external application in the field of hygiene, dermatology and dermocosmetology.

## Claims (Claims for the following Contracting State(s): ES, GR)

1. Process for preparing foams which are obtained at the time of use and which can be used in therapy, dietetics, dermocosmetology, dermatology and hygiene, characterized in that a dry powder having an extended storage life, composed of casein and an effervescent mixture, is reacted in the cold state with a diluent.

2. Process according to Claim 1, characterized in that lecithin is added to the casein, in a proportion of between 0.1 and 30 %.

3. Process according to Claim 1 or 2, characterized in that the effervescent agent consists of the combination of an acidic compound and an alkaline compound.

4. Process according to Claim 1 or 2, characterized in that the diluent is either water or a water/alcohol mixture or pure alcohol.

## Patentansprüche (Patentansprüche für folgende(n) Vertragsstaat(en): AT, BE, CH, LI, DE, DK, GB, IT, LU, NL, SE)

1. Verfahren zur Herstellung von unmittelbar vor Gebrauch hergestellten Schäumen, die auf dem Gebiet der Therapie, Diätetik, Hautkosmetik, Dermatologie und Hygiene verwendet werden können, dadurch **gekennzeichnet,** daß man ein dauerhaft konserviertes trockenes Pulver aus Casein und einer Brausemischung in der Kälte mit einem Verdünnungsmittel reagieren läßt.

2. Verfahren nach Anspruch 1, dadurch **gekennzeichnet,** daß das Casein mit Lecithin in einer Menge zwischen 0,1 und 30 % versetzt wird.

3. Verfahren nach Anspruch 1 oder 2, dadurch **gekennzeichnet,** daß das Brausemittel gebildet wird durch Zusammenlagerung einer sauren Verbindung und einer alkalischen Verbindung.

4. Verfahren nach Anspruch 1 oder 2, dadurch **gekennzeichnet,** daß das Verdünnungsmittel entweder Wasser oder ein Gemisch aus Wasser und Alkohol oder reiner Alkohol ist.

5. Präparat, das nach dem Verfahren nach einem der Ansprüche 1 bis 4 hergestellt werden kann, dadurch **gekennzeichnet,** daß es in Pulverform vorliegt.

6. Präparat nach Anspruch 5, dadurch **gekennzeichnet,** daß es in einer für die orale Verabreichung zu therapeutischen oder diätetischen Zwecken geeigneten Form vorliegt.

7. Präparat nach Anspruch 5, dadurch **gekennzeichnet,** daß es in einer Form vorliegt, die für äußere Anwendungen auf dem Gebiet der Hygiene, Dermatologie und Hautkosmetik geeignet ist.

## Patentansprüche (Patentansprüche für folgende(n) Vertragsstaat(en): ES, GR)

1. Verfahren zur Herstellung von unmittelbar vor Gebrauch hergestellten Schäumen, die auf dem Gebiet der Therapie, Diätetik, Hautkosmetik, Dermatologie und Hygiene verwendet werden können, dadurch **gekennzeichnet,** daß man ein dauerhaft konserviertes trockenes Pulver aus Casein und einer Brausemischung in der Kälte mit einem Verdünnungsmittel reagieren läßt.

2. Verfahren nach Anspruch 1, dadurch **gekennzeichnet,** daß das Casein mit Lecithin in einer Menge zwischen 0,1 und 30 % versetzt wird.

3. Verfahren nach Anspruch 1 oder 2, dadurch **gekennzeichnet,** daß das Brausemittel gebildet wird durch Zusammenlagerung einer sauren Verbindung und einer alkalischen Verbindung.

4. Verfahren nach Anspruch 1 oder 2, dadurch **gekennzeichnet,** daß das Verdünnungsmittel entweder Wasser oder ein Gemisch aus Wasser und Alkohol oder reiner Alkohol ist.
